(19) Europäisches Patentamt
European Patent Office
Office européen des brevets

(11) Veröffentlichungsnummer: **0 105 132**
**B1**

(12) # EUROPÄISCHE PATENTSCHRIFT

(45) Veröffentlichungstag der Patentschrift: **17.04.85**

(51) Int. Cl.[4]: **C 07 C 51/353**, C 07 C 53/08

(21) Anmeldenummer: **83107760.7**

(22) Anmeldetag: **06.08.83**

(54) **Katalysatorsystem und Verfahren zur Herstellung von Essigsäure durch katalytische Umlagerung von Ameisensäuremethylester.**

(30) Priorität: **01.10.82 DE 3236351**

(43) Veröffentlichungstag der Anmeldung: **11.04.84 Patentblatt 84/15**

(45) Bekanntmachung des Hinweises auf die Patenterteilung: **17.04.85 Patentblatt 85/16**

(84) Benannte Vertragsstaaten: **BE DE FR GB IT NL**

(56) Entgegenhaltungen:
**EP - A - 0 045 637**
**DE - A - 1 966 695**

(73) Patentinhaber: **CHEMISCHE WERKE HÜLS AG, - RSP Patente / PB 15 - Postfach 13 20, D-4370 Marl 1 (DE)**

(72) Erfinder: **Hög, Hans-Ulrich, Dr., Bitterfelder Strasse 9a, D-4370 Marl (DE)**
Erfinder: **Bub, Günther, Dr., Kampstrasse 94, D-4370 Marl (DE)**

**EP 0 105 132 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein Katalysatorsystem und ein Verfahren zur Herstellung von Essigsäure durch Umlagerung von Ameisensäuremethylester. Das erfindungsgemäße Katalysatorsystem enthält Rhodium, Salze oder Komplexe desselben, als weiteres Metall ein Salz oder einen Komplex mindestens eines Elements der VI. Nebengruppe sowie als Promotor ein Halogen oder eine Halogenverbindung. Die Umlagerung wird in Gegenwart von Kohlenmonoxid durchgeführt.

Die katalytische Umlagerung von Ameisensäuremethylester zu Essigsäure mit carbonylbildenden nichtedlen Metallen der VI. bis VIII. Gruppe oder deren Verbindungen in Gegenwart von Promotoren, die Vanadium, Arsen, Antimon oder Wismut sowie Halogen enthalten, wird in der DE-PS 1 072 979, der GB-PS 628 161 und der US-PS 2 508 513 beschrieben; näher ausgeführt werden Katalysatorsysteme, die Nickel oder Cobalt sowie Jod und Wismut enthalten. Diese Verfahren benötigen drastische Reaktionsbedingungen wie Temperaturen von mehr als 200 bis 335°C und Drücke von etwa 300 bar; die Essigsäureselektivitäten sind unbefriedigend, und selbst nach langer Umsetzungsdauer wird häufig nur ein mäßiger Umsatz erreicht.

Ähnlich scharfe Bedingungen werden bei Katalysatorsystemen angewendet, in denen Cobalt, Quecksilber, Eisen, Nickel oder Zink als katalytisch aktive Metalle gemeinsam mit Brom- oder Jodpromotoren und stickstoffhaltigen Lösemitteln eingesetzt werden, die in der DE-PS 2 026 031 = US-PS 3 839 428 und in den JP-ASS 30 253/73 = US-PS 3 839 428 und 16 733/75 beschrieben sind. Zwar werden dort bessere Selektivitäten für Essigsäure erreicht, jedoch sind die spezifischen Katalysatorleistungen, ausgedrückt als Gramm Essigsäure pro Gramm Metall und Stunde, nur mäßig und die Verwendung solcher Lösemittel für ein technisches Verfahren nachteilig.

Die DE-PS 2 026 031 = US-PS 3 839 428 beansprucht die Metalle der Gruppen VIII oder IIb, darunter auch Rhodium, als katalytisch aktive Komponenten. Im Beispiel 22 dieser DE-PS (bzw. Example 26 der US-PS) wird ein Katalysatorsystem aus Rhodiumchlorid, N-Methylpyrrolidon und Jodwasserstoff beschrieben. Die Konzentrationen an Rhodium-(150 mg-at Rh/mol Methylformiat), Jod-(240 mg-at J/mol Methylformiat) und Stickstoffverbindung (3,6 mol/mol Methylformiat) sind außerordentlich hoch, die Katalysatorleistung mit weniger als 1 g Essigsäure pro g Rhodium und Stunde gering. Gemäß den Ansprüchen dieser PS benötigt man die organische Stickstoffverbindung als Lösungsmittel in einer Menge von mindestens 0,2 mol pro mol Methylformiat. Der Reaktionsdruck muß insgesamt mindestens 80 bar, der Partialdruck des Kohlenmonoxides mindestens 50 bar betragen.

Lediglich im Fall des Nickels als Katalysatormetall konnten bisher gemäß der JP-OS 56-73 040 bei technisch interessanten Bedingungen wie 180°C und 50 bar Reaktionsdruck hohe Ausbeuten erzielt werden. Nachteil dieses Verfahrens ist die Notwendigkeit, die Umsetzung in Gegenwart einer organischen Stickstoffverbindung als Ligand und großer Mengen einer Jodverbindung durchzuführen. Hohe Ausbeuten von 90% und mehr werden außerdem nur bei gleichzeitigem Zusatz eines den Katalysator und das Edukt stabilisierenden Lösemittels und von Methylacetat erreicht. Nicht nur die spezifische Katalysatorleistung, die nur weniger als 15 g Essigsäure pro Gramm Nickel und Stunde erreicht, ist mäßig, sondern durch das Verdünnen mit einem Lösemittel wird auch die Raumzeitausbeute vermindert.

Die Verwendung von Rhenium- oder Rutheniumträgerkatalysatoren in einer Gasphasenreaktion, wie in der JP-OS 65 703/73 beschrieben, oder eines Palladiumkatalysators, einer Jodverbindung und einer tertiären sticktoff- oder phosphororganischen Verbindung in der Flüssigphase gemäß der JP-OS 56-22 745 führt zu deutlich schlechteren Ergebnissen als im Fall des vorstehenden Nickelkatalysatorsystems. Gemäß DE-OS 3 046 899 werden mit einem Katalysator, bestehend aus einer Palladium-, deutlich weniger gut auch Iridium- oder Ruthenium-, und einer Jodverbindung sowie einem tertiären Phosphan in Essigsäure oder Methylacetat als Lösemittel unter gleichartigen Reaktionsbedingungen ähnlich gute Ausbeuten an Essigsäure erreicht wie mit dem Nickelkatalysator. Die gleichen Nachteile wie dort treffen auch hier zu, wobei der teure Palladiumkatalysator bestenfalls eine nur geringfügig höhere spezifische Leistung zeigt.

Mit Rhodiumkatalysatoren läßt sich eine bedeutend höhere Katalysatorleistung erzielen. Nach dem Stand der Technik, wie er in der DE-PS 2 109 025 = US-PS 3 798 267 und der US-PS 4 194 056 offenbart ist, ist jedoch außer einem Jodpromotor auch der Zusatz eines Stiban-, Arsan- oder vorzugsweise Phosphanliganden notwendig, um bei der Durchführung der Reaktion unter vergleichsweise milden Bedingungen wie nicht mehr als 200°C und nicht mehr als 50 bar Kaltdruck die Stabilität des katalytisch aktiven Komplexes und eine hohe Essigsäureselektivität zu gewährleisten. Zudem sind sehr hohe Halogen/Rhodiumverhältnisse erforderlich, um eine hohe spezifische Katalysatorleistung zu erreichen. In einem Beispiel der US-PS 4 194 056 wird mit 0,84 mg-atom Rh/mol Methylformiat in Form von Rhodiumtrichlorid und Methyljodid als Promotor in dem Verhältnis J/Rh = 18,8 bei 200°C und einem Kohlenmonoxidkaltdruck von 34 bar, der zu einem Reaktionsdruck von 50 bar führt, ohne Einsatz von Phosphan in zweistündiger Reaktion ein flüssiger Austrag erhalten, der noch zu 65 Gewichtsprozent aus unumgesetztem Edukt besteht und nur 24 Gewichtsprozent Essigsäure enthält. Die spezifische Katalysatorleistung kann daraus zu höchstens 62 g Essigsäure pro Gramm Rhodium und Stunde abgeschätzt werden. In einem Beispiel der DE-PS 2 109 025 wird mit dem gleichen Katalysator, jedoch mit 0,31 mg-atom Rh/mol Mehylformiat und einem Atomverhältnis J/Rh = 258 bei 200°C und einem Kohlenmonoxidkaltdruck von 50 bar, der zu einem Reaktionsdruck von etwa 75 bar führt, in dreistündiger Reaktion ein Umsatz des Methylformiats von 99% bei einer Essigsäureausbeute von 80,4% erzielt, was einer Menge von 497 g Essigsäure pro Gramm Rhodium und Stunde entspricht. Nach einem weiteren Beispiel derselben Patentschrift erhält man mit dem Rh(I)-Komplex $[Rh(CO)_2Cl]_2$ als Rhodiumquelle unter sonst identischen Bedingungen sogar nur einen Umsatz von 43,2%, eine Essigsäureausbeute von 29,6% und damit eine Katalysatorleistung von 57 g Essigsäure pro Gramm Rhodium und Stunde.

Die unbefriedigende Wirkung von Rhodiumkatalysatorsystemen ohne Phosphanliganden geht aus den zitierten Beispielen deutlich hervor. Andererseits können solche Liganden, wenn sie bei der Kreislaufführung des Katalysators Veränderungen unterliegen, leicht zur Desaktivierung desselben führen; außerdem ist bekannt, daß derartige Liganden im allgemeinen die Aktivität eines Katalysators für Carbonylierungsreaktionen, wie zum Beispiel bei der Hydroformylierung (vgl. z. B. J. Falbe, New Synthesis with Carbon Monoxide), herabsetzen. Für die Umlagerung des Methylformiats werden in der Literatur intermediäre Carbonylierungsschritte angenommen. Um die zu erwartende Verlangsamung der Umsetzung zu vermeiden, müßten Maßnahmen wie Erhöhung der Reaktionstemperatur, der Katalysatormenge oder des Promotor/Metall-Verhältnisses ergriffen werden.

Es stellte sich daher die Aufgabe, ein Katalysatorsystem hoher Aktivität für die Umlagerung von Ameisensäuremethylester zu Essigsäure zu finden, mit dem Essigsäure in hoher Selektivität ohne Verwendung von Liganden, wie den organischen Verbindungen der Elemente der V. Hauptgruppe oder ohne zwingende Verwendung von Lösemitteln hergestellt werden kann. Vor allem zu vermeiden waren dabei selektivitätsmindernde Einflüsse, die ein Entstehen größerer Mengen an Ameisensäure bewirken, da diese die Aufarbeitung des Produktes außerordentlich erschweren würden. Mit der Bezeichnung »Umlagerung« ist keine Aussage hinsichtlich des tatsächlichen Reaktionsablaufes beabsichtigt und somit keine Einschränkung des erfindungsgemäßen Verfahrens verbunden.

Die gestellte Aufgabe wird erfindungsgemäß entsprechend den Angaben der Patentansprüche gelöst. Dazu bringt man Ameisensäuremethylester hinreichend lange, im allgemeinen 0,1 bis 10 Stunden, in flüssiger Phase bei einer erhöhten Temperatur von 140 bis 300° C in Gegenwart von Kohlenmonoxid bei einem CO-Partialdruck bei Reaktionstemperatur von 2 bis 250 bar mit einem Katalysatorsystem in Kontakt, welches Rhodium bzw. Rhodiumsalze oder Rhodiumkomplexe, mindestens eine Metallverbindung der VI. Nebengruppe und ein Halogen als Promotor, dabei jedoch keine Liganden der V. Gruppe enthält, wobei jede der notwendigen Komponenten auch als Gemisch mehrerer Elemente bzw. deren Verbindungen eingesetzt werden kann. Das Zusammenwirken der beiden metallhaltigen Komponenten zu einem Katalysatorsystem, welches in Gegenwart eines Halogenpromotors unter vergleichsweise milden Bedingungen auch ohne den Zusatz eines Lösemittels oder von Liganden die Umlagerung von Ameisensäuremethylester zu Essigsäure in ausgezeichneter Selektivität und bei hoher spezifischer Katalysatorleistung bewirkt, ist überraschend und war in keiner Weise vorauszusehen. Zwar ist schon in der JP-OS 56-73 040 der Einsatz von Chromhexacarbonyl gemeinsam mit Nickel beschrieben, doch wird dabei nur eine Essigsäureausbeute von 73,7% erreicht; ein Vorteil gegenüber Umsetzungen ohne Chromcarbonyl ist nicht erkennbar. In der US-PS 4 194 056 wird Wolfram- und Molybdänhexacarbonyl eine katalytische Wirksamkeit sogar abgesprochen.

Das Rhodium kann elementar in fein verteilter Form, als anorganisches oder organisches Salz oder als Komplexverbindung eingesetzt werden, wobei letztere keine Liganden mit Elementen der V. Hauptgruppe enthält. Dabei ist es vorteilhaft, daß sich aus so einfachen Verbindungen wie $RhCl_3 \cdot 3\,H_2O$, die eine ausgezeichnete Wirksamkeit für das Verfahren zeigen, unter Reaktionsbedingungen die aktive Katalysatorform ohne spezielle Präformierung bildet. Als Metallverbindung der VI. Nebengruppe sind Chrom- und Molybdänverbindungen bevorzugt; besonders geeignet sind die Halogenide und/oder Carbonyle; insbesondere sind Chromcarbonyl und/oder Chromhalogenide geeignet. Die Metallverbindungen können auch auf Träger aufgebracht eingesetzt werden. Als Träger sind beispielsweise Aktivkohle, Kieselgel und/oder Aluminiumoxid geeignet. Als Halogenpromotoren eignen sich Brom und Jod als Element oder in Form einer Verbindung. Geeignete Einsatzformen umfassen Verbindungen der Form $X_3^-$ und HX mit X = Br oder J, anorganische und organische Bromide und Jodide. Als anorganische Verbindungen können Salze derjenigen Metalle dienen, die die Reaktion ihrerseits nicht ungünstig beeinflussen, wie zum Beispiel Bromide und Jodide der Alkali- und Erdalkalimetalle, gegebenenfalls unter Einhalten einer Präformierphase in Gegenwart einer geringen Menge, bevorzugt etwa der gleichen Molmenge des anorganischen Promotors, an Wasser, oder auch der Katalysatormetalle selbst, als organische Verbindungen Alkyl-, Acyl- und Arylbromide und -jodide. Bevorzugt sind Jod und seine Verbindungen, insbesondere Methyljodid.

Zusätzliche Liganden wie tertiäre Phosphane sind nicht erforderlich und bringen auch keine Vorteile. Lösemittel zur Stabilisierung des Methylformiats sind nicht erforderlich, sondern wirken sogar unter Umständen eher schädlich (Vergleichsbeispiel d). Der Zusatz von Essigsäure bringt jedoch keine Nachteile (Beispiel 12). Lösemittel und Essigsäure ermöglichen jedoch die Umsetzung bei niederen Drücken (Beispiel 23). Es ist vorteilhaft, praktisch wasserfrei zu arbeiten, da die Gegenwart von Wasser die Verseifung des Ameisensäureesters (Gl. 1) oder in Gegenwart des Produktes Essigsäure die Transesterifizierung (Gl. 2) fördert; dieser Effekt kann durch die thermische Zersetzung der gebildeten Ameisensäure (Gl. 3) verstärkt werden, und zu einem unerwünschten Druckanstieg im Reaktionsgefäß führen.

$$HCOOCH_3 + H_2O \longrightarrow HCOOH + CH_3OH \qquad \text{(Gl. 1)}$$

$$HCOOCH_3 + CH_3COOH \xrightarrow{[H_2O]} HCOOH + CH_3COOCH_3 \qquad \text{(Gl. 2)}$$

$$HCOOH \xrightarrow{\Delta T} H_2O + CO \qquad \text{(Gl. 3)}$$

$$HCOOH \xrightarrow{[Kat]} H_2 + CO_2 \qquad \text{(Gl. 4)}$$

Aus Methanol und Methylacetat ihrerseits können sich, vor allem wenn sich auch Wasserstoff zum Beispiel gemäß Gl. 4 bildet, weitere Nebenprodukte bilden, deren Gegenwart die Aufarbeitung erschwert. Auch Ameisensäure selbst im Produktgemisch führt zu derartigen Schwierigkeiten. Wassermengen im Reaktionsgemisch von nicht mehr als 5 Gewichtsprozent, günstiger von nicht mehr als 2 Gewichtsprozent, können jedoch geduldet werden. Dabei ist es wegen des Herstellungsverfahrens für Ameisensäuremethylester unproblematisch, das Edukt praktisch wassrfrei zu erhalten. In Abwesenheit von Wasser ist außerdem die Korrosion des Reaktormaterials durch die produzierte Essigsäure und das Halogen des Promotors stark vermindert, so daß gegebenenfalls auf teure Sondermaterialien verzichtet werden kann. Weiter ist es vorteilhaft, daß das Produkt in praktisch wasserfreier Form anfällt.

Außer in der Flüssigphase mit suspendiertem oder homogen gelösten Katalysator kann das Verfahren zwar auch in der Gasphase durchgeführt werden, wobei zum Beispiel die metallhaltigen Katalysatorbestandteile in fester Form oder auf einen Träger wie Aktivkohle, Kieselgel oder Aluminiumoxid aufgebracht mit einem gasförmigen Einsatzgemisch aus dem Edukt, einer flüchtigen Jod- oder Bromverbindung und Kohlenmonoxid in Kontakt gebracht werden. Umsatz und/oder Selektivität sind jedoch beim Gasphasenverfahren ungünstiger als in der Flüssigphase. Das Verfahren läßt sich kontinuierlich oder diskontinuierlich durchführen.

Nach beendeter Umsetzung kann das Produkt destillativ abgetrennt werden; flüchtige Jodverbindungen werden dabei als Vorlauf abgenommen und gemeinsam mit dem bei Durchführung in der Flüssigphase den Katalyator enthaltenen Destillationssumpf in den Reaktor zurückgeführt. Das erfindungsgemäße Verfahren ist jedoch auf eine bestimmte Ausprägung der technischen Ausführung nicht beschränkt.

Die Reaktion erfolgt in Gegenwart von Kohlenmonoxid. Dieses wird zwar gemäß der Stöchiometrie für die Umsetzung nicht benötigt, dient aber zur Stabilisierung des Ameisensäureesters und der aktiven Katalysatorform gegen Zersetzung und nötigenfalls zur Bildung dieser aktiven Katalysatorform aus den eingesetzten Verbindungen. Das Gas kann, gegebenenfalls unter Abzweigen eines kleinen Abfallstroms zum Ausschleusen gasförmiger Verunreinigungen, im Kreis geführt werden. Verunreinigungen des Kohlenmonoxids durch Stickstoff, Methan, Kohlendioxid, Wasserstoff, wie beispielsweise im Synthesegas, oder andere inerte Gase stören auch in größerer Menge nicht, sollten aber gering gehalten werden, um zur Einstellung des notwendigen CO-Partialdrucks keinen unerwünscht hohen Reaktionsdruck zu benötigen. Bei Verwendung von reinem Kohlenmonoxid wird das Verfahren bei Reaktionsdrücken von 2 bis 250 bar, bevorzugt bei 25 bis 100 bar, durchgeführt. Typische CO-Kaltdrükke im bevorzugten Bereich bei einer Durchführung der Reaktion in der Flüssigphase sind 5 bis 80 bar. Der zur Stabilisierung des Eduktes oder des Katalysators vorzulegende Mindestdruck an Kohlenmonoxid ist von der Reaktionstemperatur abhängig und kann bei Bedarf schnell und einfach ermittelt werden.

Die Umsetzungstemperatur des Verfahrens beträgt 140 bis 300°C, bevorzugt 160 bis 220°C. Die Reaktionsdauer kann in Abhängigkeit von den übrigen Verfahrensparametern in weiten Grenzen variieren und wird zweckmäßigerweise so eingerichtet, daß ein praktisch vollständiger Umsatz erreicht wird. Eine Reaktionsdauer von zwei Stunden und weniger läßt sich dabei ohne weiteres erreichen.

Es ist leicht einzusehen, daß der Einsatz einer möglichst geringen Menge an Katalysator, vor allem des Rhodiums als dem kostspieligsten Bestandteil, wirtschaftlich wünschenswert ist. Daher ist es von Vorteil, daß das Verfahren im Vergleich zu Katalysatorsystemen, die auf anderen Metallen aufbauen, mit sehr geringem Katalysatoreinsatz auskommt. Geeignet sind Mengen an Rhodium oder Rhodiumverbindung, die 0,05 bis 5 mg-atom Rhodium pro mol Ameisensäuremethylester, bevorzugt 0,1 bis 1 mg-atom/mol entsprechen. Dabei beträgt das Atomverhältnis Rhodium/Halogen, bevorzugt Rh/J, 1 : 1000 bis 1 : 1, bevorzugt 1 : 100 bis 1 : 5, und das Atomverhältnis Rhodium/Metall der VI. Nebengruppe, bevorzugt Rh/Cr, 1 : 100 bis 10 : 1, bevorzugt 1 : 20 bis 2 : 1. Es können zwar grundsätzlich auch größere Mengen jeder Kompomente eingesetzt werden, jedoch bewirkt das keine Verbesserung und ist wirtschaftlich ungünstig. Bei kleineren Mengen dagegen ist es notwendig, in unerwünschter Weise die Umsetzungsdauer zu verlängern oder die Reaktionsbedingungen zu verschärfen.

Das erfindungsgemäße Verfahren ermöglicht es, Essigsäure in hoher Reinheit durch katalytische Umlagerung von Ameisensäuremethylester bei hoher spezifischer Katalysatorleistung praktisch quantitativ als alleiniges Produkt zu erhalten. Man erreicht Katalysatoraktivitäten von 1520 g freie Essigsäure/g Rh · h (Beispiel 10). Die Katalysatoraktivität und -selektivität bleibt auch nach destillativem Abtrennen des Produktes und der Rückführung des Katalysators voll erhalten (Beispiel 13). Im folgenden wird das Verfahren durch Beispiele näher erläutert.

## Beispiel 1

In einem 100 ml-Autoklaven aus Hastelloy C werden 41 g (683 mmol) Methylformiat, 0,2 g (0,76 mmol) Rhodiumtrichlorid-trihydrat, 1,6 g (7,2 mmol) Chromhexacarbonyl und 5 g (35,2 mmol) Methyljodid vorgelegt. Der Autoklav wird druckfest verschlossen und mit Kohlenmonoxid gespült. Anschließend wird Kohlenmonoxid bis 35 bar aufgepreßt, auf 180°C aufgeheizt, der Reaktionsdruck mittels geringer CO-Zugabe auf 50 bar eingestellt und die Umsetzung unter intensivem Rühren zwei Stunden lang durchgeführt. Danach erfolgt schnelles Abkühlen (15 min) durch Einblasen von Preßluft in die Heizung und Entspannen des Autoklaven über einen Abgaswäscher. Nach Zusatz definierter Mengen 1,4-Dioxan als innerem Standard werden der flüssige Reaktionsaustrag und der Wäscherinhalt gaschromatographisch analysiert; es zeigt sich, daß das Methylformiat fast quantitativ zur gewünschten Essigsäure abreagiert ist. Die Geschwindigkeit der Umsetzung ist hoch; die spezifische Katalysatoraktivität wird mit 260 g Essigsäure/g Rh · h berechnet. Als einziges Nebenprodukt wird eine geringe Menge Methylacetat gefunden; Ameisensäure und Methanol können nicht nachgewiesen werden. Wasser ist im flüssigen Austrag in kleiner Menge (25 mmol) enthalten. Das aufgefangene Abgas besteht fast ausschließlich aus Kohlenmonoxid. Wasserstoff, Kohlendioxid und Methan können nicht oder nur in geringen Mengen von wenigen mMol nachgewiesen werden.

## Vergleichsbeispiele a bis d

Diese Beispiele betreffen nicht das erfindungsgemäße Verfahren. Die Vergleichsbeispiele werden entsprechend Beispiel 1 durchgeführt; abweichende Versuchsbedingungen, Einsatzmengen und Ergebnisse sind in Tabelle 1 zusammengefaßt. Man erkennt, daß bei verschiedenen Versuchsbedingungen Umsatz, Selektivität und Katalysatoraktivität stets weitaus schlechter sind, wenn $Cr(CO)_6$ als Katalysatorbestandteil fehlt. Auch ein Zusatz von Triphenylphosphan gleicht diesen Nachteil nicht völlig aus. Vergleichsbeispiel d zeigt darüber hinaus, daß im Gegensatz zu den Nickelkatalysatorsystemen der JP-OS 56-73 040 die Gegenwart eines Lösemittels beim Rh/Cr/J-System nicht nur unnötig, sondern unter Umständen sogar nachteilig ist.

Als Nebenprodukte werden Methylacetat, Ameisensäure meist in dazu stöchiometrisch äquivalenter Menge, etwas Methanol und Acetaldehyd und zum Teil nennenswerte Mengen nicht identifizierter Verbindungen gebildet. Die Mengen an $H_2$, $CH_4$ und $CO_2$ im Abgas sind höher als im Beispiel 1 (Ergebnisse in Tabelle 1).

## Beispiele 2 bis 9

Die Versuche werden entsprechend Beispiel 1 durchgeführt; abweichende Versuchsbedingungen, Einsatzmengen und Ergebnisse sind in Tabelle 1 zusammengefaßt. Sie zeigen, daß nur kleine Katalysatormengen notwendig sind, um die Umsetzung mit hoher Geschwindigkeit und Selektivität ablaufen zu lassen; dabei kann eine unter Umständen bei geringer Rhodium- oder Promotorkonzentration zu beobachtende unerwünschte Verminderung dieser Größen unschwer durch mäßige Steigerung der Temperatur behoben werden. Senkt man beispielsweise die Methyljodidmenge gegenüber Beispiel 5 auf ein Viertel, so sinken Umsatz und Selektivität auf jeweils ein Fünftel, um bei Steigerung der Reaktionstemperatur auf 200°C wieder den Vergleichswerten zu entsprechen. Die anzuwendenden Konzentrationen des Halogenpromotors sind vergleichsweise niedrig; auch die Metallverbindung der VI. Nebengruppe wird nur in der geringen Größenordnung des Rhodiumkatalysators benötigt. Der Reaktionsdruck kann ohne weiteres so weit vermindert werden, daß er nur wenig über dem Dampfdruck des Methylformiats allein bei der betreffenden Temperatur liegt. Es werden Katalysatoraktivitäten von über 500 g Essigsäure pro Gramm Rhodium und Stunde bei 99% Ausbeute erzielt. Im Fall von $Mo(CO)_6$ an Stelle des $Cr(CO)_6$ wird ebenfalls ein besseres Ergebnis erreicht als ohne diese Komponente; für einen vollständigen Umsatz in derselben Zeit von zwei Stunden wäre jedoch eine etwas höhere Reaktionstemperatur oder Promotormenge nötig.

Tabelle 1

| Bei-spiel Nr. | $HCOOCH_3$ (g) | Katalysator (g) | Pro-motor (g) | weitere Kat.-bestandteile (g) | Rh/Cr/J/P*) (Atomverh.) | p**) (bar) | T (°C) | t (h) | U (%) | S(mol-%)***) AcOH | AcOMe | Kat. akt.****) (g AcOH/ g Rh · h) |
|---|---|---|---|---|---|---|---|---|---|---|---|---|
| 1 | 41 | $RhCl_3 \cdot 3\,H_2O$ (0,2) | $CH_3J$ (5,0) | $Cr(CO)_6$ (1,6) | 1/9,5/46/— | 50 | 180 | 2 | 99,6 | 99,7 | 0,1 | 260 |
| 2 | 41 | $RhCl_3 \cdot 3\,H_2O$ (0,1) | $CH_3J$ (2,5) | $Cr(CO)_6$ (0,8) | 1/9,5/46/— | 50 | 200 | 2 | 99,3 | 99,7 | Spur | 519 |
| 3 | 41 | $RhCl_3 \cdot 3\,H_2O$ (0,2) | $CH_3J$ (5,0) | $Cr(CO)_6$ (1,6) | 1/9,5/46/— | 35 | 180 | 2 | 95,6 | 99,3 | — | 249 |
| 4 | 41 | $RhCl_3 \cdot 3\,H_2O$ (0,2) | $CH_3J$ (5,0) | $Cr(CO)_6$ (1,6) | 1/9,5/46/— | 35 | 170 | 2 | 97,9 | 92,7 | 3,6 | 238 |
| 5 | 41 | $RhCl_3 \cdot 3\,H_2O$ (0,2) | $CH_3J$ (5,0) | $Cr(CO)_6$ (0,8) | 1/4,7/46/— | 50 | 180 | 2 | 99,8 | 99,8 | Spur | 261 |
| 6 | 41 | $RhCl_3 \cdot 3\,H_2O$ (0,2) | $CH_3J$ (5,0) | $Cr(CO)_6$ (0,17) | 1/1/46/— | 50 | 180 | 2 | 99,5 | 94,8 | 1,3 | 247 |
| 7 | 41 | $RhCl_3 \cdot 3\,H_2O$ (0,2) | $CH_3J$ (2,5) | $Cr(CO)_6$ (1,6) | 1/9,5/23/— | 50 | 180 | 2 | 99,1 | 99,3 | 0,5 | 258 |
| 8 | 41 | $RhCl_3 \cdot 3\,H_2O$ (0,2) | $CH_3J$ (2,5) | $Cr(CO)_6$ (0,8) | 1/4,7/23/— | 35 | 180 | 2 | 87,9 | 89,7 | 7,8 | 207 |
| 9 | 41 | $RhCl_3 \cdot 3\,H_2O$ (0,2) | $CH_3J$ (5,0) | $Mo(CO)_6$ (0,95) | 1/4,7/46/— | 50 | 180 | 2 | 51,6 | 90,3 | 10,2 | 122 |

*) Cr steht für das Element der VI. Nebengruppe.
**) Reaktionsdruck; Kaltdruck 35 bar, außer Vers. 4 (20 bar), Vers. 5, 9 (5,5 bar) und Vgl.-Vers. b (50 bar).
***) gemäß Reaktionsstöchiometrie, d. h. 2 Mol $HCOOCH_3$ je Mol AcOMe.
****) nur freie AcOH.

Tabelle 1 (Fortsetzung)

| Vgl.-Bsp. Nr. | $HCOOCH_3$ (g) | Katalysator (g) | Promotor (g) | weitere Kat.-bestandteile (g) | Rh/Cr/J/P*) (Atomverh.) | p**) (bar) | T (°C) | t (h) | U (%) | S(mol-%)***) AcOH | AcOMe | Kat. akt.****) (g AcOH/ g Rh · h) |
|---|---|---|---|---|---|---|---|---|---|---|---|---|
| a | 41 | $RhCl_3 \cdot 3\,H_2O$ (0,2) | $CH_3J$ (0,5) | — | 1/—/46/— | 50 | 180 | 2 | 27,0 | 58,1 | 21,1 | 41 |
| b | 30 | $RhCl_3 \cdot 3\,H_2O$ (0,0414) | $CH_3J$ (5,77) | — | 1/—/259/— | 78 | 200 | 3 | 27,3 | 38,6 | 28,6 | 65 |
| c | 41 | $RhCl_3 \cdot 3\,H_2O$ (0,2) | $CH_3J$ (5,0) | $PPh_3$ (0,4) | 1/—/46/2 | 50 | 180 | 2 | 68,5 | 93,1 | 6,7 | 167 |
| d | 20,5 | $RhCl_3 \cdot 3\,H_2O$ (0,1) | $CH_3J$ (2,5) | $Cr(CO)_6$ (0,8) + Acetophenon (30) | 1/9,5/46/— | 50 | 180 | 2 | 64,0 | 87,2 | — | 146 |

*) Cr steht für das Element der VI. Nebengruppe.
**) Reaktionsdruck; Kaltdruck 35 bar, außer Vers. 4 (20 bar), Vers. 5, 9 (5,5 bar) und Vgl.-Vers. b (50 bar).
***) gemäß Reaktionsstöchiometrie, d. h. 2 Mol $HCOOCH_3$ je Mol AcOMe.
****) nur freie AcOH.

Beispiel 10

Die Umsetzung erfolgt nach den Angaben des Beispiels 1, jedoch werden nur 34 mg $RhCl_3 \cdot 3\,H_2O$ und 267 mg $Cr(CO)_6$ eingesetzt und die Reaktion bei 200° C und 50 bar durchgeführt. Bei mehrfachem Wiederholen des Versuches unter den genannten Bedingungen werden Methylformiat-Umsätze von 92 bis 99,5% und Essigsäure-Selektivitäten von 87 bis 99% gemessen. Als weiteres Produkt wird bei den höheren Umsätzen nur Methylacetat, bei den niedrigeren Umsätzen zusätzlich Ameisensäure gefunden. Die Katalysatoraktivitäten betragen zwischen 1190 und 1520 g freier Essigsäure pro g Rhodium und Stunde.

Beispiel 11

Die Umsetzung erfolgt nach den Angaben des Beispiels 1, jedoch werden statt des $RhCl_3 \cdot 3\,H_2O$ äquivalente Mengen $Rh_4(CO)_{12}$ bzw. $[Rh(CO)_2Cl]_2$ eingesetzt. Es werden die gleichen Ergebnisse wie in Beispiel 1 erzielt.

Beispiel 12

Man arbeitet nach den Angaben des Beispiels 1, setzt jedoch 10 g Essigsäure zusätzlich ein. Umsatz und Selektivität zu neugebildeter Essigsäure sind quantitativ.

Beispiel 13

Man arbeitet nach den Angaben des Beispiels 1, engt jedoch den Reaktionsaustrag destillativ im schwachen CO-Strom auf etwa 10 ml ein und setzt den Destillationssumpf gemeinsam mit frischem Methylformiat (41 g) und Methyljodid (5 g) unter gleichen Bedingungen wieder in die Reaktion ein. Der Methylformiat-Umsatz ist quantitativ, wobei Methylformiat ausschließlich zu Essigsäure umgesetzt wird; das Katalysatorsystem bleibt also durch die Destillation unbeeinträchtigt.

Beispiel 14

Man arbeitet nach den Angaben des Beispiels 1, setzt jedoch 0,96 g $CrCl_3 \cdot 6\,H_2O$ statt der dort angegebenen 1,6 g $Cr(CO)_6$ ein. Es werden die gleichen Ergebnisse wie in Beispiel 1 erzielt.

Beispiel 15

Wiederholt man Beispiel 5, indem man das Chromcarbonyl durch eine äquivalente Menge Chrom-(III)-acetylacetonat ersetzt, beträgt der Umsatz 99% und die Essigsäure-Selektivität 96 mol-%; der Rest besteht praktisch ausschließlich aus Methylacetat. Bei Verminderung der Menge an Chromverbindung bis auf ein Cr/Rh-Verhältnis von 1 : 1 sinken Umsatz und Selektivität unter sonst unveränderten Bedingungen zwar auf jeweils 70 bis 75%, wobei als weitere Produkte nur Methylacetat und eine äquivalente Menge Ameisensäure neben einer Spur Methanol gebildet werden (vgl. Beispiel 6 mit Chromcarbonyl).

Diese Ergebnisse sind aber weiterhin um ein Mehrfaches besser als die der Vergleichsbeispiele a und b ohne Chromkomponente.

Beispiel 16

Wird die Umsetzung in gleicher Weise wie in Beispiel 15, jedoch mit einer äquivalenten Menge $Cr_3(OH)_2(CH_3COO)_7$ durchgeführt, so beträgt der Umsatz ebenfalls 99%. Neben geringen Mengen Methylacetat und Ameisensäure ($\leq$1 mol-%) bildet sich nur Essigsäure. Die Beispiele 14 bis 16 belegen, daß auch andere Chromverbindungen anstelle des Chromcarbonyls wirksam sind.

Beispiel 17

Die Umlagerung von Methylformiat (41 g) wurde in Gegenwart von $RhCl_3 \cdot 3\,H_2O$ (0,2 g), $LiJ \cdot 2\,H_2O$ (2,99 g) und $Cr(CO)_6$ (0,8 g), was einem Verhältnis Rh/Cr/J von 1 : 4,7 : 23 entspricht, bei 190°C und 50 bar Heißdruck unter CO-Atmosphäre durchgeführt. Nach zwei Stunden war der Ester zu 99%

umgesetzt; Essigsäure war mit 85 mol-% Selektivität entstanden. Der Rest bestand aus gleichen molaren Mengen Methylacetat und Ameisensäure. Dieses Beispiel zeigt, daß der organische Jodpromotor vollständig durch einen anorganischen ersetzbar ist.

## Beispiel 18

Bei Durchführung der Reaktion entsprechend Beispiel 17, jedoch mit einer äquivalenten Menge NaJ als Promotor und bei nur 180°C wird ein Umsatz von 45% und eine Selektivität für Essigsäure von 31 mol-% erreicht. Das restliche Produkt besteht aus Methylacetat und Ameisensäure. Eine leichte Verbesserung ist bei Gegenwart einer kleinen Menge Wasser zu beobachten. Da ohne Jodverbindung praktisch kein Umsatz erfolgt, ist auch hier eine Promotorwirkung festzustellen. In diesem Fall muß jedoch zum Erzielen eines hohen Umsatzes zu Essigsäure entweder die Reaktionstemperatur erhöht, die Umsetzungsdauer verlängert oder eine Präformierung vorgeschaltet werden.

## Vergleichsbeispiele

Wird die Umsetzung wie in Beispiel 18, jedoch mit einer entsprechenden Menge wäßriger 57prozentiger HJ als Jodverbindung durchgeführt, so werden nur 15% Umsatz erreicht, obwohl HJ unter Reaktionsbedingungen praktisch quantitativ in Methyljodid überführt wird. Essigsäure entsteht nur mit 7% Selektivität. Hauptprodukte sind Ameisensäure und Methylacetat neben deutlichen Mengen an Methanol und Acetaldehyd. Das Vergleichsbeispiel veranschaulicht den nachteiligen Einfluß größerer Mengen Wasser; eine Eignung wasserfreier HJ als Promotor soll damit nicht verneint werden. In einem Experiment entsprechend Beispiel 1 bewirkt der Zusatz von 5 g Wasser zwar nur eine Verminderung der Essigsäureausbeute von > 99 auf 93%, begleitet von 3% Ameisensäure, doch ist ein Druckanstieg im Autoklaven von 50 auf 85 bar und vermehrte Bildung von $H_2$, $CH_4$ und $CO_2$ zu beobachten.

## Beispiel 19

Wiederholt man Beispiel 5, indem man das Methyljodid durch die entsprechende Menge Jod ersetzt, so daß das Atomverhältnis Rh/J unverändert bleibt, erhält man eine quantitative Umsetzung zu Essigsäure; Nebenprodukte sind weder im Flüssigprodukt noch im Abgas zu finden. Das eingesetzte Jod findet sich überwiegend in Form von Methyljodid (29 mmol) wieder.

## Beispiel 20

Wiederholt man Beispiel 5, jedoch mit 367,5 mg $RhJ_3$ als Rhodiumkomponente und nur 4,68 g Methyljodid, so erhält man eine quantitative Umsetzung zu Essigsäure ohne nachweisbare Nebenprodukte. Das Beispiel zeigt, daß der Promotor auch mit dem Metallkatalysator eingeführt werden kann.

## Beispiel 21

Bei Wiederholung des Beispiels 5, jedoch mit einer gleichen molaren Menge Methylbromid als Promotor und bei 200°C, wird ein Umsatz von 44% und Selektivitäten von 58 mol-% für Essigsäure und 38 mol-% für Methylacetat, begleitet von einer entsprechenden Menge Ameisensäure, erzielt.

In geringen Mengen werden Methanol und im Abgas $H_2$ und $CH_4$ gefunden. Das Beispiel zeigt die Promotorwirkung einer Bromverbindung, wenngleich diese schwächer ist als die der analogen Jodverbindung.

## Beispiel 22

Beispiel 5 wird wiederholt, indem man das bisher verwendete Kohlenmonoxid durch Synthesegas ($CO/H_2 = 1$) ersetzt und ebenfalls bei 50 bar Heißdruck arbeitet. Trotz der Gegenwart einer großen Menge Wasserstoffs und der Halbierung des Partialdrucks des Kohlenmonoxids erhält man vollständigen Umsatz zu Essigsäure. Eine Metallabscheidung durch Reduktion des Katalysators wird nicht beobachtet.

Beispiel 23

Der Ansatz aus Beispiel 3 wird halbiert und durch Zugabe von 20,5 g Essigsäure ergänzt. Ein Kaltdruck von 5 bar CO wird aufgepreßt und auf 180°C aufgeheizt, wobei sich ein Heißdruck von 12 bar einstellt. Bei diesen Bedingungen wird die Reaktion wie in Beispiel 3 durchgeführt. Man erhält bei > 99% Umsatz eine Selektivität für Essigsäure von 97,5 mol-%; der Rest ist Methylacetat. Das Beispiel veranschaulicht eine Maßnahme, um den Reaktionsdruck abzusenken. Die Zugabe von Essigsäure ist zu diesem Zeck am günstigsten, da sie als das herzustellende Produkt die Aufarbeitung nicht aufwendiger macht. Grundsätzlich eignen sich aber auch andere Carbonsäuren und darüber hinaus beliebige Lösemittel, sofern sie nicht wie im Vergleichsbeispiel d das Katalysatorsystem nachteilig beeinflussen. Die im Einzelfall notwendige Eignungsprüfung kann nach der Lehre der vorliegenden Schrift in einfacher Weise erfolgen.

Beispiel 24

Die Umsetzung wird mehrfach gemäß Beispiel 5 durchgeführt, jedoch zu verschiedenen Zeitpunkten beendet. Nach 30, 60 bzw. 90 Minuten beträgt der Umsatz 12, 63 bzw. praktisch 100%. Die Reaktion verläuft in diesem Zeitraum mit rund 25% Eduktumsatz je 15 Minuten mit konstanter Geschwindigkeit und ist in 80 bis 85 Minuten beendet. Darauf bezogen beträgt die Katalysatorleistung um 380 g Essigsäure je g Rhodium und Stunde.

Die Selektivität für Essigsäure beträgt nach den genannten Zeiten 20, 95 bzw. fast 100 mol-%. Weiterhin findet man an Methylacetat 21, 17 bzw. 0,5 mmol, an Ameisensäure 19,5 und 15 mmol bzw. keine erkennbaren Mengen mehr. Andere Substanzen sind nicht oder nur in Spuren vorhanden, speziell Methanol und im Abgas $H_2$ und $CH_4$. Der Wassergehalt bewegt sich mit wenigen Zehntel Gewichtsprozent im Bereich des Feuchtigkeitsgehaltes des ohne besondere Vorreinigung eingesetzten Ausgangsmaterials.

Die geringere Reaktionsgeschwindigkeit während der ersten 30 Minuten kann auf die Bildung einer nicht bekannten aktiven Katalysatorform während dieses Zeitraums zurückzuführen sein. Tatsächlich ist die Anfangsgeschwindigkeit der Reaktion höher, wenn man z. B. $[Rh(CO)_2Cl]_2$ als Rh-Komponente einsetzt (33% Umsatz in 30 Minuten bei 84 mol-% Essigsäureselektivität).

Beispiel 25

Wiederholt man Beispiel 5 mit sorgfältig absolutierten Einsatzmaterialien, wobei in diesem Fall eine äquivalente Menge $[Rh(CO)_2Cl]_2$ anstelle des $RhCl_3 \cdot 3\,H_2O$ eingesetzt wird, um auch die geringe Menge Kristallwasser auszuschließen, erzielt man eine besonders hohe Anfangsgeschwindigkeit von 460 mmol Essigsäure fast ausschließlich in freier Essigsäure in 30 Minuten. Das Beispiel zeigt den Vorteil des Wasserausschlusses bei unserem Verfahren.

Beispiel 26

Führt man Beispiel 8 bei 50 bar Reaktionsdruck durch, so erhält man bei 97,8% Umsatz Essigsäure mit > 95% Selektivität. Dieses Beispiel zeigt, daß nötigenfalls eine Verbesserung der Umsetzung auch durch mäßige Druckerhöhung zu erreichen ist.

**Patentansprüche**

1. Verfahren zur Herstellung von Essigsäure durch katalytische Umlagerung von Ameisensäuremethylester in Gegenwart eines Metallkatalysators, eines Halogenpromotors und von Kohlenmonoxid bei erhöhter Temperatur und erhöhtem Druck, dadurch gekennzeichnet, daß man die Umsetzung in der Flüssigphase in Abwesenheit von Liganden der V. Hauptgruppe an einem Metallkatalysatorsystem aus Rhodium bzw. Rhodiumsalzen oder Rhodiumkomplexen und aus mindestens einer Metallverbindung der VI. Nebengruppe in Gegenwart eines Halogenpromotors bei Temperaturen von 140 bis 300°C und CO-Partialdrücken bei diesen Reaktionstemperaturen von 2 bis 250 bar durchführt, wobei man das Rhodium oder die Rhodiumverbindung in Mengen von 0,05 bis 5 mg-atom Rhodium pro mol eingesetztes Methylformiat einsetzt und ein Atomverhältnis Rhodium : Metall der VI. Nebengruppe von 1 : 100 bis 10 : 1 und des Rhodiums : Halogen von 1 : 1000 bis 1 : 1 einhält.

2. Verfahren nach Anspruch 1, dadurch gekennzeichnet, daß man als Metallverbindung der VI. Nebengruppe ein Metallcarbonyl und/oder ein Metallhalogenid, vorzugsweise Chromcarbonyl und/oder Chromhalogenid, einsetzt.

3. Verfahren nach den Ansprüchen 1 und 2, dadurch gekennzeichnet, daß man als Halogenpromotoren Brom, Jod oder Verbindungen von Brom und/oder Jod, bevorzugt Methyljodid, einsetzt.

4. Verfahren nach den Ansprüchen 1 bis 3, dadurch gekennzeichnet, daß man das Rhodium bzw. die Rhodiumverbindung in Mengen von 0,1 bis 1 mg-atom Rhodium pro mol eingesetztes Methylformiat einsetzt.

5. Verfahren nach den Ansprüchen 1 bis 4, dadurch gekennzeichnet, daß man in dem Metallkatalysatorsystem ein Atomverhältnis Rhodium : Metall der VI. Nebengruppe von 1 : 20 bis 2 : 1 und des Rhodiums : Halogen von 1 : 100 bis 1 : 5 einhält.

6. Verfahren nach den Ansprüchen 1 bis 5, dadurch gekennzeichnet, daß man die Umsetzung bei einer Temperatur von 160 bis 220° C und Reaktionsdrücken von 25 bis 100 bar durchführt.

7. Katalysatorsystem zur Herstellung von Essigsäure durch Umlagerung von Ameisensäuremethylester nach den Ansprüchen 1 bis 6, dadurch gekennzeichnet, daß das Katalysatorsystem aus Rhodium bzw. einer Rhodiumverbindung, die keine Liganden der V. Hauptgruppe enthält, mindestens einer Metallverbindung der VI. Nebengruppe, bevorzugt Chromcarbonyl und/oder ein Chromhalogenid und einem Halogenpromotor, bevorzugt Methyljodid, besteht, wobei das Atomverhältnis Rhodium : Metall der VI. Nebengruppe 1 : 100 bis 10 : 1 und das des Rhodiums : Halogen 1 : 1000 bis 1 : 1 beträgt.

## Claims

1. A process for the production of acetic acid by catalytic rearrangement of methyl formate at elevated temperature and elevated pressure in the presence of a metal catalyst, a halogen promoter and carbon monoxide, characterised in that the reaction is carried out in the liquid phase at a temperature of 140 to 300° C and a CO partial pressure of 2 to 250 bar at the reaction temperature by means of a metal catalyst system comprising rhodium, a rhodium salt or a rhodium complex and at least one metal compound of Group VI B in the presence of a halogen promoter but in the absence of ligands of Group V A, the rhodium compound being supplied in an amount of 0.05 to 5 mg atom of rhodium per mole of methyl formate supplied and at an atomic ratio of rhodium: Group VI B metal of 1 : 100 to 10 : 1 and an atomic ratio of rhodium : halogen of 1 : 1000 to 1 : 1.

2. A process according to claim 1, characterised in that a metal carbonyl and/or a metal halide, preferably chromium carbonyl and/or a chromium halide, is supplied as Group VI B metal compound.

3. A process according to claim 1 or 2, characterised in that bromine, iodine or a compound of bromine and/or of iodine, preferably methyl iodide, is supplied as halogen promoter.

4. A process according to claim 1, 2 or 3, characterised in that the rhodium or rhodium compound is supplied in an amount of 0.1 to 1 mg atom of rhodium per mole of methyl formate supplied.

5. A process according to any of claims 1 to 4, characterised in that an atomic ratio of rhodium: Group VI B metal of 1 : 20 to 2 : 1 and an atomic ratio of rhodium : halogen of 1 : 100 to 1 : 5 is used in the metal catalyst system.

6. A process according to any of claims 1 to 5, characterised in that the reaction is carried out at a temperature of 160 to 220° C and a reaction pressure of 25 to 100 bar.

7. A catalyst system for use in the production of acetic acid by rearrangement of methyl formate according to any of claims 1 to 6, characterised in that the catalyst system comprises rhodium or a rhodium compound which contains no ligands of Group V A, at least one metal compound of Group VI B, preferably chromium carbonyl and/or a chromium halide, and a halogen promoter, preferably methyl iodide, the atomic ratio of rhodium : Group VI B metal being 1 : 100 to 10 : 1 and the atomic ratio of rhodium : halogen 1 : 1000 to 1 : 1.

## Revendications

1. Procédé de préparation d'acide acétique par transposition catalytique du fomiate de méthyle en présence d'un catalyseur métallique, d'un activeur halogéné et de monoxyde de carbone, à température élévée et à pression élévée, caractérisé par le fait que l'on conduit la réaction en phase liquide en l'absence de coordinats du groupe principal V, sur un système catalytique métallique formé de rhodium ou de sels de rhodium ou de complexes de rhodium et d'au moins un composé métallique du groupe secondaire VI, en présence d'un activeur halogéné, à des températures de 140 à 300° et à des pressions partielles de CO de 2 à 250 bar à ces températures de réaction, en utilisant le rhodium ou le composé de rhodium à raison de 0,05 à 5 milliatomes-grammes de rhodium par mole de formiate de méthyle mis en œuvre et que l'on maintient un rapport atomique rhodium/métal du groupe secondaire VI compris entre 1 : 100 et 10 : 1 et un rapport atomique rhodium/halogène compris entre 1 : 1000 et 1 : 1.

2. Procédé selon la revendication 1, caractérisé par le fait que comme composé de métal du groupe secondaire VI, on utilisé un carbonyl-métal et/ou un halogénure métallique, de préférence le carbonyl-chrome et/ou un halogénure de chrome.

3. Procédé selon les revendications 1 et 2, caractérisé par le fait que comme activeurs halogénés, on utilise le brome, l'iode ou des composés de brome et/ou d'iode, de préférence l'iodure de méthyle.

4. Procédé selon les revendications 1 à 3, caractérisé par le fait que l'on utilise le rhodium ou le composé de rhodium à raison de 0,1 à 1 milliatome-gramme de rhodium par mole de formiate de méthyle utilise.

5. Procédé selon les revendications 1 à 4, caractérisé par le fait que l'on maintient dans le système de catalyseur métallique un rapport atomique rhodium/métal du groupe secondaire VI compris entre 1 : 20 et 2 : 1 et un rapport atomique rhodium/halogène compris entre 1 : 100 et 1 : 5.

6. Procédé selon les revendications 1 à 5, caractérisé par le fait que l'on effectue la réaction à une température de 160 à 220° C et à des pressions de réaction de 25 à 100 bar.

7. Système catalytique pour la préparation d'acide acétique par transposition du formiate de méthyle selon les revendications 1 à 6, caractérisé par le fait que le système catalytique est formé de rhodium ou d'un composé de rhodium qui ne contient pas de coordinats du groupe principal V, d'au moins un composé de métal du groupe secondaire VI, de préférence le carbonyl-chrome et/ou un halogénure de chrome et d'un activeur halogéné, de préférence l'iodure de méthyle, le rapport atomique rhodium/métal du groupe secondaire VI étant compris entre 1 : 100 et 10 : 1 et le rapport atomique rhodium/halogène étant compris entre 1 : 1000 et 1 : 1.